# EUROPÄISCHE PATENTSCHRIFT

(11) **EP 1 390 139 B1**
(45) Veröffentlichungstag und Bekanntmachung des Hinweises auf die Patenterteilung: **26.04.2006**
(21) Anmeldenummer: 02753706.7
(22) Anmeldetag: 27.02.2002
(51) Int. Cl.: B01J 19/00

(54) **VERFAHREN ZUR HERSTELLUNG EINES ARRAYS ZUR DETEKTION VON KOMPONENTEN AUS EINER BIOLOGISCHEN PROBE**
METHOD FOR PRODUCING AN ARRAY FOR DETECTING CONSTITUENTS FROM A BIOLOGICAL SAMPLE
PROCEDE POUR PRODUIRE UN JEU ORDONNE D'ECHANTILLONS POUR DETECTER DES CONSTITUANTS DANS UN ECHANTILLON BIOLOGIQUE

(30) Priorität: 28.02.2001 DE 10110511
(43) Veröffentlichungstag der Anmeldung: 25.02.2004
(73) Patentinhaber: Attomol GmbH Molekulare Diagnostika, 03205 Lipten (DE)
(72) Erfinder: LEHMANN, Werner, 03205 Lipten (DE)
(74) Vertreter: Ziebig, Marlene
(86) Internationale Anmeldenummer: PCT/EP2002/002116
(87) Internationale Veröffentlichungsnummer: WO 2002/076608

(56) Entgegenhaltungen:
- EP-A- 0 238 190
- WO-A-00/40942
- WO-A-02/05945
- WO-A-96/17246
- WO-A-99/13313
- WO-A-99/19711
- ELLIOTT P. DAWSON ET AL.: "MEMBRANE-BASED MICROARRAYS" PROCEEDINGS OF THE SPIE, SPIE, Bd. 3857, September 1999 (1999-09), Seiten 31-37, XP000997211 BELLINGHAM, VA, US

## Beschreibung

Die Erfindung betrifft ein Verfahren zur Herstellung eines Arrays zur Detektion von Komponenten aus einer biologischen Probe. Das Verfahren eignet sich insbesondere zur Herstellung eines kompakten Arrays, da die Detektionsmoleküle des Arrays mit einer sehr hohen Dichte auf den Array aufgebracht werden können.

In der klinischen Diagnostik und Lebensmittelanalytik werden seit mehreren Jahren immobilisierte Detektionsmoleküle, z.B. Enzyme und Antikörper, als Erkennungssubstanzen für verschiedene Komponenten in biologischen Proben eingesetzt. Werden viele dieser Detektionsmoleküle gleichzeitig auf einen festen Träger aufgebracht, so spricht man von einem Array bzw. von einem Mikroarray.

Mit Arrays können viele Proben gleichzeitig und bei geringem Material- und Arbeitsaufwand untersucht werden. Bekannte Arrays bestehen zum Beispiel aus vielen mikroskopischen Spots, von denen jeder identische Detektionsmoleküle, beispielsweise einzelsträngige Oligonucleotide, cDNA bzw. Antikörper, enthalten kann, die auf einem festen Träger wie Glas oder einem Polymer fixiert sind. Bei der Fixierung von einzelsträngigen Oligonucleotiden oder cDNA enthält jeder Spot viele Kopien einer besonderen Sequenz, die 10-2000 Basen aufweisen können.

Bei der Detektion oder Analyse erfolgt eine Wechselwirkung mit der Probe, wobei sich z.B. ein Doppelstrang mit einer komplementären Sequenz bildet. Es handelt sich hierbei um die sogenannte Hybridisierung. Wenn die Spots der Detektionsmoleküle beispielsweise Kopien von bestimmten Antikörpern enthalten, erfolgt die Analyse oder Detektion durch Wechselwirkung mit den Antigenen einer biologischen Probe, wobei sich Antigen-Antikörperkomplexe bilden.

Von großer Bedeutung bei dem Einsatz von Arrays ist die Dichte der aufgebrachten und immobilisierten Detektionsmoleküle, beispielsweise DNAs, Antikörper und/oder Rezeptoren.

Mitteldichte Arrays weisen 100 bis 1000 verschiedene immobilisierte Detektionsmoleküle auf, die sogenannten hochdichten Arrays verfügen über ca. 10.000 immobilisierte Moleküle. Insbesondere bei der Herstellung der sogenannten hochdichten Chips bzw. Arrays treten im Hinblick auf eine Massenproduktionsfähigkeit mehrere Nachteile auf. Diese sind beispielsweise die komplizierte Immobilisierungsprozedur und das kostenaufwendige Herstellungsverfahren. Ein weiterer Nachteil der bisherigen Verfahren zur Herstellung von Arrays bzw. Mikroarrays ist, dass die Arrays, insbesondere für klinische Anwendungen, nicht mit der erforderlichen Reproduzierbarkeit hergestellt werden können. Das heißt, Arrays für Routineuntersuchungen, die z.B. mit gleichen Antikörpern oder Rezeptoren beschichtet werden, unterscheiden sich untereinander von ihrem Aufbau und der Dichte der aufgebrachten immobilisierten Moleküle, wodurch die Untersuchungen nicht oder schwer vergleichbar sind.

Um dieses Ziel zu erreichen wurden verschiedene Lösungswege eingeschlagen. So beschreibt WO 01/09607 A1 das Arrangement fibrillärer Träger, die in einem Einbettungsmedium eingebettet werden, um senkrecht zur Fibrillenlängsausdehnung dünne Schnitte herstellen zu können, die jeweils identische Anschnitte aller Fibrillen aufweisen und als Array dienen. Das Arrangement vieler Fibrillen ist jedoch kompliziert in der Ausführung und außerdem können die Materialien der Fibrillen die spätere Analyse von Biomolekülen negativ beeinflussen. Die Analyse komplexer Proteingemische ist nur schwer zu realisieren. Um komplexe Verteilungsmuster von Biomolekülen in biologischen Geweben abbilden zu können, wurde ein Gewebearray (US 4914022) patentiert. Dafür werden Proben ausgewählter Gewebe gemeinsam in einem Paraffinblock eingebettet, geschnitten und die Schnitte auf einen Träger aufgebracht. Mit dieser Erfindung können zwar die Verteilungsmuster in verschiedenen Gewebeschnitten untersucht werden, eine Zuordnung auf molekularer Ebene wie z.B. in einem Western-Blot nach 1D- oder 2D-Auftrennung von Proteingemischen ist jedoch nicht möglich.

Der Erfindung liegt daher die Aufgabe zugrunde, ein Verfahren zur Herstellung eines Arrays zur Detektion von Komponenten aus einer biologischen Probe bereitzustellen, mit dem Arrays kostengünstig, einfach und mit guter Reproduzierbarkeit auch bei hoher Komplexität der Probe hergestellt werden können.

Die vorliegende Erfindung löst dieses technische Problem durch die Bereitstellung eines Verfahrens zur Herstellung eines Arrays zur Detektion von Komponenten aus einer biologischen Probe, wobei die Detektionsmoleküle auf einem ersten Träger immobilisiert werden, mindestens ein erster Träger in einem Material eingebettet wird, das Material zum Aushärten gebracht wird, der eingebettete erste Träger senkrecht in Schnitte separiert wird, mindestens ein Schnitt auf einen zweiten Träger so aufgebracht wird, dass die Detektionsmoleküle den zweiten Träger kontaktieren und von dem aufgebrachten Schnitt mit einem Lösungsmittel das ausgehärtete Material und/oder der erste Träger vollständig oder teilweise auf- oder abgelöst werden und der Array erhalten wird.

Arrays im Sinne der Erfindung können spezifische Membrankomponenten sein, die den Stoffdurchtritt durch Biomembranen ermöglichen, erleichtern bzw. beschleunigen. Im Sinne der Erfindung sind Arrays aber auch unlösliche organische und anorganische, makromolekulare Stoffe, wie zum Beispiel poröses Glas, PVDF-Membranen und ähnliches, mit denen lösliche Komponenten, wie zum Beispiel Enzyme, aber auch Mikroorganismen, verbunden werden können, um über diese Komponenten Stoffe detektieren zu können.

Eine biologische Probe im Sinne der Erfindung ist jedes Material, dass aus Mikroorganismen, Pflanzen, Tieren oder dem Menschen gewonnen wurde. Biologische Proben im Sinne der Erfindung umfassen daher Blut, Serum, Chromosomen, Föten, Gewebeproben, Gameten, Urin, Stuhl, Tränenflüssigkeit, Sekrete, Atemluft, Schweißabsonderungen, Schleimhautabstriche, Knochenmaterial, Knorpel, Haare, Proteine, Nucleotidsequenzen, Kohlenhydrate, Lymphe, Darminhalt, entartetes Gewebe, Zellen, Zellbruchstücke, DNA, RNA, Lipide und ähnliches. Eine biologische Probe im Sinne der Erfindung können jedoch auch entnommene Teilmengen aus Abwässern, Industrieprozess rückständen, Mooren oder anderen Umweltflüssigkeiten sein. Eine Probe im Sinne der Erfindung ist daher jedes durch Probenentnahme entnommene Gut oder ein Teil bzw. eine kleine Menge eines solchen, dessen Beschaffenheit physikalisch, chemisch und/oder biologisch geprüft werden soll.

Detektionsmoleküle gemäß der Erfindung sind alle Substanzen, mit denen Komponenten aus einer biologischen Probe bestimmt oder detektiert werden können. Wenn in einer biologischen Probe beispielsweise antigene Strukturen untersucht werden sollen, sind die Detektionsmoleküle im Sinne der Erfindung beispielsweise Antikörper. Detektionsmoleküle können jedoch auch Rezeptoren sein, die mit Signalmolekülen in der biologischen Probe wechselwirken. Bei den Detektionsmolekülen kann es sich jedoch auch um DNA- bzw. RNA-Abschnitte handeln, die mit DNA- und RNA-Abschnitten in der biologischen Probe hybridisieren. Detektionsmoleküle im Sinne der Erfindung können jedoch auch Mikroorganismen, Zellen oder Zellextrakte sein.

Ein Träger im Sinne der Erfindung ist ein Material, an das lösliche Detektionsmoleküle gebunden werden können.

Unter Immobilisierung im Sinne der Erfindung sind alle Methoden zur Einschränkung der Beweglichkeit und Löslichkeit von Detektionsmolekülen auf chemischen, biologischen und/oder physikalischen Wegen zu verstehen. Die Immobilisierung kann durch unterschiedliche Methoden erfolgen, wie der Bindung der Detektionsmoleküle untereinander oder an Träger, durch Festhalten im Netzwerk einer polymeren Matrix oder Umschließen durch Membranen. Durch die Immobilisierung werden die Detektionsmoleküle nicht nur wiederverwendbar, sondern können nach dem Prozess der Interaktion mit der biologischen Probe leicht wieder abgetrennt werden. Sie lassen sich in sehr viel höheren lokalen Konzentrationen und in kontinuierlichen Durchflusssystemen einsetzen. Die Bindung bzw. die Immobilisierung der Detektionsmoleküle an den Träger kann durch direkte Trägerverbindung und durch Quervernetzung erfolgen. Die Trägerbindung bzw. Quervernetzung erfolgt gemäß der Erfindung insbesondere ionisch/adsorptiv oder durch kovalente Bindung. Die Quervernetzung im Sinne der Erfindung ist eine Vernetzung der Detektionsmoleküle untereinander oder mit anderen Polymeren. Bei der Immobilisierung durch Einschluß werden die Detektionsmoleküle in Gelstrukturen bzw. in Membranen eingeschlossen.

Gemäß dem erfindungsgemäßen Verfahren zur Herstellung eines Arrays werden die Detektionsmoleküle auf einem planaren oder nicht planaren ersten Träger immobilisiert. Bei dem ersten Träger handelt es sich um eine Membran, beispielsweise um eine Nitrozellulosemembran. Auf den ersten Träger können Detektionsmoleküle z.B. durch Auftropfen von runden Spots oder durch Aufdrucken von Linien immobilisiert werden.

Als erste Träger können planare wie auch nicht planare Gebilde wie Kugeln, Quader oder Fibrillen und Stäbe eingesetzt werden, die mit unterschiedlichen Detektionsmolekülen imprägniert und anschließend in eine einbettbare und schneidbare räumliche Anordnung gebracht werden.

Dem Fachmann sind zahlreiche Möglichkeiten bekannt, die Detektionsmoleküle auf dem ersten Träger zu immobilisieren. Die Immobilisierung sollte hierbei so erfolgen, dass jeder Sonde eine definierte Position auf dem Träger zugeordnet werden kann und dass jede Position auf dem ersten Träger unabhängig ausgewertet werden kann. Es kann aber auch gewünscht sein, dass sich die Auftragungsorte verschiedener Sonden teilweise oder vollständig überlappen oder dass Sondengemische aufgetragen werden. Die Immobilisierung kann beispielsweise mit einem an die Halbleitertechnik angelehnten Verfahren erfolgen. Im wesentlichen können die Detektionsmoleküle auf zwei prinzipiell verschiedenen Wegen auf dem ersten Träger immobilisiert werden: Zum einen ist die In situ-Synthese der Detektionsmoleküle an definierten Positionen auf dem ersten Träger durch sukzessive Kopplung monomerer Synthesebausteine möglich, zum anderen ist ein Ablegen und Immobilisieren von zuvor synthetisierten oder aus Bibliotheken stammenden Detektionsmolekülen an definierten Positionen des insbesondere funktionalisierten Trägermaterials möglich. Hierfür können sowohl Spotting als auch Druckverfahren eingesetzt werden. Unter Spotting versteht man Verfahren, bei denen Flüssigkeitstropfen, in denen die Detektionsmoleküle befindlich sind, auf dem Träger abgelegt werden, wobei durch Oberflächenwechselwirkung und Trocknung im wesentlichen runde Spots entstehen. Aber auch andere Druckverfahen ermöglichen es, die Detektionsmoleküle in definierten Flächen auf der Oberfläche des ersten Trägers abzulegen, wodurch eine stabile Bindung der Proben an die Substratoberfläche der Detektionsmoleküle mit hoher Kopplungseffizienz erfolgen kann. Alle dem Fachmann bekannten Immobilisierungsmaßnahmen von Biomolekülen an z.B. Säulenmaterialien können ebenfalls verwandt werden, um die Detektionsmoleküle auf dem ersten Träger zu immobilisieren.

Ausgewählte Verfahren zum Immobilisieren sind beispielsweise das Contact Tip Printing, Ring and Pin Printing, Nanoelectric Printing and Nanopipetting, Buble Jet Printing, TopSpot Printing, Micro Contact Printing, Micro Fluidic Networks-Methoden, Photolithographic Activation-Verfahren, Photoresist Lithography, Electrochemical Focusing und Micro Wet Printing. Es können aber auch vergleichsweise einfache Probenauftragsgeräte aus der Dünnschichtchromatographie oder Autosampler für die HPLC eingesetzt werden. Mit Hilfe dieser Verfahren kann eine Spot-Größe von unter einem Mikrometer bis über 1000 Mikrometer erzeugt werden. Die Sondendichte pro cm² kann erfindungsgemäß beispielsweise im Bereich von ca. 1 bis 1 Million liegen. Die genannten Verfahren ermöglichen eine einfache Handhabung und eine große Präzision der angelegten Volumina sowie sehr homogene Spots, die mit einer hohen Parallelisierung auftragen werden können. Die Verfahren, die eine direkte Synthese der Detektionsmoleküle auf dem ersten Träger ermöglichen, weisen insbesondere eine hohe Integrationsdichte und eine einfache Kombination von Immobilisierung und Hybridisierung auf. Es ist möglich, die Detektionsmoleküle, die auch als Sonden bezeichnet werden können, mit weiteren Verfahren vorzubereiten, um den Anteil unspezifisch synthetisierter bzw. gebundener Detektionsmoleküle gering zu halten.

Die Detektionsmoleküle können jedoch auf dem ersten Träger auch durch ein Blot-Verfahren immobilisiert werden. Hierzu werden Detektionsmoleküle, wie beispielsweise Antikörper, Rezeptoren, Gewebeextrakte, Mikroorganismen und ähnliches, mittels einer Elektrophorese aufgetrennt. Die elektrophoretische Auftrennung kann z.B. mittels einer eindimensionalen oder mehrdimensionalen Elektrophorese, insbesondere einer 2D-Elektrophorese, erfolgen.

Die elektrophoretisch aufgetrennten bzw. separierten Detektionsmoleküle werden dann entweder auf den ersten Träger übertragen bzw. auf eine Membran, die in einem nächsten Schritt auf den ersten Träger aufgebracht wird.

Erfindungsgemäß wird mindestens ein erster Träger, auf dem die Detektionsmoleküle immobilisiert werden, in einem Material eingebettet. Dem Fachmann sind zahlreiche biologische und chemische Einbettungsmaterialien bekannt. Das Material sollte chemisch weitestgehend inert sein, damit es mit den Detektionsmolekülen nicht so in Wechselwirkung treten kann, dass diese nachteilig modifiziert werden. Es kann jedoch auch vorgesehen sein, die Detektionsmoleküle durch die Wechselwirkung mit dem Material zu modifizieren. Es ist selbstverständlich möglich, auf die Einbettung zu verzichten, falls der oder die ersten Träger eine schneidbare Konsistenz aufweisen. D.h., wenn die Konsistenz des oder der Träger es erlauben, sie ohne Behandlung zu schneiden, müssen die oder der Träger nicht eingebettet werden.

Das Material kann beispielsweise aus gesättigten Kohlenwasserstoffen bestehen und bei Raumtemperatur einen festen Aggregatzustand aufweisen. Das Material kann weiterhin so beschaffen sein, dass es mit üblichen Labormethoden, beispielsweise durch Erwärmen oder Ultraschallbehandlung, verflüssigt werden kann. Mit Hinblick auf den einzubettenden Träger mit den immobilisierten Detektionsmolekülen sollte das Material seinen flüssigen Aggregatzustand auch bei Temperaturen von unter 60° Grad Celsius beibehalten. Oberhalb dieser Temperatur beginnen bestimmte Proteine und Peptide bzw. Gewebe- und/oder Mikroorganismenextrakte zu denaturieren.

In einem weiteren Verfahrensschritt des erfindungsgemäßen Verfahrens wird das Material zum Aushärten gebracht. Das Aushärten kann beispielsweise durch einfaches Abkühlen des Materials erfolgen oder durch Zugabe von chemischen Substanzen, die ein Aushärten des Materials initiieren. Es ist jedoch auch möglich, das Material durch physikalische Einwirkungen wie Ultraschall oder UV- bzw. Röntgenstrahlung auszuhärten. Das Aushärten des Materials sollte so erfolgen, dass der erste Träger und die Detektionsmoleküle nicht nachteilig verändert werden. Durch das Aushärten des Materials wird der Träger in seiner eingebetteten Lage stabilisiert und kann dadurch beispielsweise mechanisch, chemisch oder biologisch bearbeitet werden.

Der/die eingebettete(n) erste(n) Träger wird/werden in einem weiteren Schritt des erfindungsgemäßen Verfahrens senkrecht in Schnitte separiert. Wenn die ersten Träger aufgrund ihrer Konsistenz ohne Behandlung, wie beispielsweise dem Einbetten, in Schnitte separiert werden können, erfolgt das Separieren selbstverständlich an den nicht eingebetteten Trägern. Wenn die Detektionsmoleküle auf dem Träger in Form mehrerer Linien immobilisiert sind, können die Schnitte so separiert werden, dass Schnitte entstehen, auf denen mehrere Spots von immobilisierten Detektionsmolekülen, die Teilbereiche der separierten Linien darstellen, vorhanden sind. Die einzelnen Spots auf dem Schnitt weisen dann selbstverständlich den gleichen Abstand zueinander auf wie die Linien auf dem ersten Träger.

In einem weiteren Schritt des erfindungsgemäßen Verfahrens wird mindestens ein Schnitt auf einen zweiten Träger so aufgebracht, dass die Detektionsmoleküle den zweiten Träger kontaktieren. Es kann beispielsweise vorgesehen sein, dass mehrere Schnitte, beispielsweise nebeneinander, auf dem zweiten Träger aufgebracht werden. Schnitte, die von ein und demselben ersten Träger separiert wurden, können eine nahezu identische Struktur der aufgebrachten Detektionsmoleküle aufweisen. Wenn mehrere dieser gleichen Schnitte auf ein- und demselben zweiten Träger aufgebracht werden, sind insbesondere klinische Routine- und Screening-Untersuchungen mit entsprechenden Vergleichs- und Kontrollmöglichkeiten effizient möglich. Eine besonders vorteilhafte Einsatzmöglichkeit sind differentialdiagnostische Untersuchungen.

Der oder die Schnitte werden dabei so auf den zweiten Träger aufgebracht, dass die Detektionsmoleküle mit dem zweiten Träger verbunden sind oder den zweiten Träger kontaktieren. Das Aufbringen des Schnittes auf dem zweiten Träger kann durch einfaches Auflegen erfolgen, wobei die Adhäsions- und Kohäsionskräfte die Fixierung des Schnittes auf dem zweiten Träger positiv beeinflussen können. Das kann z.B. durch Auflegen des Schnittes auf einen Flüssigkeitstropfen und Erwärmen zum Strecken der Schnitte erreicht werden. Es kann jedoch auch vorgesehen sein, dass spezielle Substanzen oder Vorrichtungen, die dem Fachmann bekannt sind, verwendet werden, um einen oder mehrere Schnitte auf dem zweiten Träger aufzubringen und in einer gewünschten Position zu fixieren. Der zweite Träger kann beispielsweise ein Objektträger aus Glas sein, der waagerecht positioniert ist, so dass ein oder mehrere Schnitte auf ihn aufgelegt werden können. Die Schnitte können aber z.B. auch auf den Boden einer Mikrotestplatte oder auf eine Membran aufgebracht werden.

Von den aufgebrachten Schnitten werden gemäß dem erfindungsgemäßen Verfahren mit einem Lösungsmittel das ausgehärtete Material und/oder der erste Träger vollständig oder teilweise abgelöst, wodurch der Array erhalten wird. Durch das vollständige oder teilweise Ablösen des ersten Trägers und/oder des ausgehärteten Materials werden insbesondere die Detektionsmoleküle an den zweiten Träger gebunden. Dem Fachmann sind verschiedene Lösungsmittel bekannt, um ausgehärtetes Material oder den ersten Träger vollständig oder teilweise abzulösen. Das Lösungsmittel sollte so beschaffen sein, dass die Detektionsmoleküle in ihrer dreidimensionalen Struktur und Funktionsfähigkeit weitestgehend erhalten bleiben. Das Lösungsmittel kann insbesondere so ausgewählt werden, dass es möglich ist, die Detektionsmoleküle zu stabilisieren und wenn erforderlich, auch über Monate, mit möglichst hoher Aktivität unter unterschiedlichsten Belastungen haltbar zu machen. Durch das Lösen des ersten Trägers und/oder des Materials werden die Detektionsmoleküle so auf dem zweiten Träger fixiert, dass ein wiederholter Einsatz unter technischen Bedingungen möglich ist. Die Detektionsmoleküle können von dem zweiten Träger durch Adsorption, Ionenbindung oder kovalent gebunden werden. Wenn die Detektionsmoleküle Enzyme, Proteine, Nukleinsäuren, Kohlenhydrate, Lipide etc. sind, kann dies auch innerhalb der ursprünglich mikrobiellen, pflanzlichen oder tierischen Zellen bzw. Viren stattfinden. Durch die Bindung der Detektionsmoleküle an den zweiten Träger können sie mehrfach und kontinuierlich eingesetzt werden.

Es ist auch möglich, dass die Bindung an den Träger durch Einschluss erfolgt, wenn beispielsweise der zweite Träger eine semipermeable Membran in Form von Gelen, Mikropartikeln oder Fasern ist. Die so eingeschlossenen Detektionsmoleküle sind dann durch eine semipermeable Membran von der umgebenden biologischen Probe getrennt. Die Detektionsmoleküle werden insbesondere durch die Fixierung im Raum mittels Einschluss nicht in ihrer Aktivität beeinflußt. Der Einschluss der Detektionsmoleküle ist beispielsweise auch darin möglich, wenn die zweiten Träger Sinterglasschwämme oder Blotmembranen sind, wodurch auch eine Aufkonzentrierung der Detektionsmoleküle möglich ist. Diese Aufkonzentrierung kann z.B. durch Kapillarblotting mittels Flüssigkeitsstrom oder Elektroblotting erfolgen. Die Aufkonzentrierung oder der Transfer der Detektionsmoleküle kann auch durch Auflösen des ersten Trägers auf dem zweiten Träger erfolgen. Vorzugsweise kann dieses Auflösen so gestaltet werden, dass das Lösungsmittel im Semi-dry-Verfahren bevorzugt den zweiten und ersten Träger von unten durchtränkt. Das Lösungsmittel kann aber auch von oben als Aerosol aufgetragen werden, wobei erster und zweiter Träger von oben durchtränkt werden. Weiterhin ist eine Immobilisierung auf dem zweiten Träger durch Geleinschluss Carageen in Alginat oder in ENT-Polymeren möglich, durch den Einschluss in Polyacrylamid, oder auf Keramik und auf keramischen Trägern mit Polyamin und/oder mittels der Quervernetzung durch Glutaraldehyd möglich.

Weiterhin ist es möglich, dass der zweite Träger als Ultrafiltrationsmembran ausgebildet ist, hinter oder vor der eingeschlossene Detektionsmoleküle positioniert sind.

Wenn eine möglichst große Oberfläche für die Adsorption oder kovalente Bindung erhalten werden soll, werden makroporöse Träger bevorzugt. Eine mögliche Voraussetzung für die kovalente Fixierung der Detektionsmoleküle ist die Anwesenheit von funktionellen Gruppen auf dem Träger. Ein mögliches Aktivierungsverfahren, beispielsweise bei Dextrangelen, ist die Umsetzung mit Bromcyan. Entsprechend der chemischen Natur der funktionellen Gruppen können sich so verschiedene Bindungstypen ausbilden, zum Beispiel Ether, Thioether, Ester usw. Dem Fachmann sind weiterhin Kopplungsverfahren zur kovalenten Anknüpfung von Detektionsmolekülen an Agar-, Agarose- und Sephadexträger sowie an silanisierte Oberflächen von porösen Gläsern bekannt. Mögliche Änderungen der Aktivität der Detektionsmoleküle können umgangen oder reduziert werden, indem die Detektionsmoleküle über Spacer an dem zweiten Träger immobilisiert werden, wobei dem Fachmann bekannt ist, dass die Behandlung von erstem und zweitem Träger identisch oder verschieden erfolgen kann. Die Spacer verleihen den Detektionsmolekülen insbesondere mehr Beweglichkeit und ermöglichen damit ungehinderten Kontakt mit der biologischen Probe. Das mögliche Vernetzen von Detektionsmolekülen durch bi- oder multifunktionale Spacermoleküle führt dazu, dass mehr Detektionsmoleküle am zweiten Träger immobilisiert werden können. Es ist beispielsweise möglich, dass die Spacer dem Lösungsmittel zur vollständigen oder teilweisen Ablösung des ausgehärteten Materials und/oder dem ersten Träger zugesetzt werden. Selbstverständlich können die Detektionsmoleküle auch an dem ersten Träger über Spacer immobilisiert werden.

Wenn die Detektionsmoleküle mittels einer 2D-Elektrophorese aufgetrennt werden, können je nach Verlauf der einzelnen insbesondere punktförmig ausgebildeten Spots die separierten Schnitte diese Spots aufweisen bzw. sie nicht aufweisen. Wenn ein Spot beispielsweise einen Durchmesser von einem Millimeter aufweist und Schnitte im Bereich von einem zehntel Millimeter separiert werden, so würden ungefährt 10 Schnitte den Spot aufweisen. Wenn angestrebt wird, dass die zweiten Träger jeweils alle Spots des ersten Trägers aufweisen sollen, so ist es erforderlich, dass nach Beendigung des Schneidens des ersten Spots mit Beginn des Schneidens des folgenden nächsten Spots die entsprechenden Schnitte auf den zweiten Träger immobilisiert werden, auf denen sich bereits Schnitte des vorausgegangenen Spots befinden, aufweisen. Es kann jedoch selbstverständlich auch vorgesehen sein, dass die zweiten Träger nicht alle Spots des ersten Trägers aufweisen, sondern jeweils nur einzelne Spots des ersten Trägers umfassen.

In einer bevorzugten Ausführungsvariante der Erfindung werden mindestens zwei erste Träger überlappend in dem Material eingebettet. Vorteilhafterweise können durch das Überlappen von mindestens zwei ersten Trägern sehr kompakte Arrays hergestellt werden. Beispielsweise ist es möglich, zwei oder mehrere erste Träger mit linienförmig angeordneten Detektionsmolekülen übereinander zu positionieren. Die Dichte der Detektionsmoleküle beruht zum einen auf dem Abstand der Linien auf jeweils einem ersten Träger und zum anderen auf der Packungsdichte der eingebetteten ersten Träger untereinander. Werden die ersten Träger beispielsweise sehr dünn gewählt, zum Beispiel Membranen, ist eine sehr dichte Packung von mehreren Membranen übereinander möglich. Mit Vorteil können die ersten Träger so ausgewählt werden, dass möglichst viele Detektionsmoleküle auf der Oberfläche positioniert werden können, zum Beispiel mit Hilfe eines Druckers. Zweckmäßigerweise müssen Stabilitätsforderungen, denen jeder Array genügen muss, wenig berücksichtigt werden, da der eigentliche zum Einsatz kommende Träger der zweite Träger ist. Der zweite Träger kann insbesondere nach Stabilitätskriterien oder anderen Kriterien; die beispielsweise im klinischen Routinebetrieb wichtig sind, ausgewählt werden.

Es ist beispielsweise möglich, 50, 100 oder mehr erste Träger, beispielsweise sehr dünn ausgebildete Membranen, übereinander zu stapeln und in dem Material einzubetten. Durch ein dichtes linienförmiges oder punktförmiges Aufbringen von vielen Detektionsmolekülen und eine große Anzahl von übereinander überlappend angeordneten ersten Trägern, wird so eine sehr hohe Dichte der Detektionsmoleküle erzielt. Die überlappend angeordneten ersten Träger werden dann senkrecht, z.B. zu den in Linien aufgebrachten Detektionsmolekülen, geschnitten. Auf dem Schnitt befinden sich dann zum einen die Detektionsmoleküle der untereinander angeordneten Träger, aber auch einzelne Spots der jeweils auf einem Träger angeordneten Detektionsmoleküle, wodurch sehr kompakte Arrays gebildet-werden können.

In einer weiteren vorteilhaften Ausführungsvariante der Erfindung werden als Detektionsmoleküle Mikroorganismen, Zellen, Zellextrakte, Liganden, Antigene, Antikörper, Rezeptoren, Nukleinsäuren, Lektine, Proteine, Peptide, Glycopeptide, Kohlenhydrate und/oder Lipide eingesetzt. Bei den Mikroorganismen kann es sich um lebende oder tote Mikroorganismen handeln, wobei die lebenden Mikroorganismen im Sinne der Erfindung sowohl wachsende als auch ruhende Zellen betreffen. Mikroorganismen können beispielsweise durch Adhäsion und Bewuchs auf dem ersten Träger immobilisiert werden. Die passive Immobilisierung, die zu einem mikrobiellen Bewuchs führt, bildet einen Biofilm auf dem ersten Träger. Am Zustandekommen der Adhäsion sind außer van-der-Waals-Kräften noch andere Bindungskräfte wie beispielsweise hydrophobe Wechselwirkungen, Wasserstoffbrückenbindungen und ionische Bindungen beteiligt. Die Adhäsionsfähigkeit hängt von der chemischen Zusammensetzung, Gesamtladung und dem Alter der Mikroorganismen ab sowie von der Ladung, Zusammensetzung aber auch der Porösität des ersten Trägers. Als erste Träger für Mikroorganismen können insbesondere Glas, Titan- und Zirkondioxid, Cellulose, Nitrozellulose, Nylon und Polyvinylalkohol-Membranen eingesetzt werden. Die Mikroorganismen können auch durch intrazelluläre und interzelluläre Vernetzung der Zellen auf dem ersten Träger immobilisiert werden. Bei dem Einschluss in eine Polymermatrix zur Immobilisierung der Mikroorganismen werden zweckmäßigerweise Biopolymere, wie beispielsweise Polysaccharide oder Proteine oder aber synthetische Polymere, insbesondere Polyacrylamid, verwendet, wobei z.B. Biopolymerperlen entstehen. Diese Biopolymerperlen, in denen die Zellen eingeschlossen sind, werden entweder durch Chelatisierung oder durch Quervernetzung - beispielsweise mit Glutardealdehydfixiert. Bei den Einschlussverfahren zur Immobilisierung von Mikroorganismen sind die Poren des ersten Trägers vorteilhafterweise kleiner als die Mikroorganismen. Die Mikroorganismen verbleiben somit innerhalb des Einschlusses, während Substrate und Produkte aus der biologischen Probe ein- bzw. austreten können. Es ist jedoch auch möglich, die Mirkoorganismen auf dem ersten Träger durch Einkapselung in Membranen zu immobilisieren. Hierbei kann im Sinne der Erfindung zwischen der Einkapselung in feste und flüssige Membranen unterschieden werden. Feste Membranen werden beispielsweise aus einer vorgefertigten Membran, zum Beispiel in Membranreaktoren, wie Hohlfasermembran-Reaktoren, gebildet, oder aber die Membran wird unmittelbar um die Zellsuspension herum ausgebildet, wie dies beispielsweise bei der Mikroverkapselung der Fall ist. Flüssige Membranen werden beispielsweise durch die Phasengrenze zwischen zwei nichtmischbaren Flüssigkeiten gebildet. Die Funktionsfähigkeit bzw. der physiologische Zustand der Mikroorganismen wird durch die Immobilisierung vorteilhafterweise nur gering beeinflusst. Immobilisierte Mikroorganismen weisen beispielsweise die Vorteile der leichteren Zellabtrennung und der Zellrückhaltung auf. Vorteilhaft ist weiterhin, dass Enzyme in den Mikroorganismen in ihrem natürlichen Mikromilieu zweckmäßigerweise eine höhere pH-, Temperatur- und Operationsstabilität besitzen. Vorteilhafterweise sind Multienzymreaktionen deshalb leichter durchführbar als mit immobilisierten Enzymen. Entsprechendes gilt insbesondere für Coenzym-abhängige Reaktionen mit in-Situ-Regenerierung der Coenzyme/Cofaktoren.

Der Einsatz von immobilisierten Mikroorganismen ist insbesondere dann von Vorteil, wenn (i) die an der Stoffumwandlung beteiligten Enzyme intrazellulär lokalisiert sind, (ii) die aus den Zellen isolierten Enzyme während und nach der Immobilisierung inshabil sind, (iii) die Mikroorganismen keine interferierenden Enzyme enthalten und/oder (iv) die Enzyme leicht inaktiviert oder entfernt werden können sowie (v) die Substrate und Produkte ein geringes Molekulargewicht besitzen. Selbstverständlich ist es auch möglich, die Mikroorganismen mit Enzymen zusammen auf dem ersten Träger zu co-immobilisieren. Die Co-Immobilisierung im Sinne der Erfindung ist die Immobilisierung intakter, lebender oder toter Zellen von Mikroorganismen mit freien oder immobilisierten Enzymen; zum Beispiel können immobilisierte Enzyme mit ganzen Zellen in eine gemeinsame Matrix eingeschlossen werden oder die Enzyme direkt an lebende Zellen gekoppelt werden, die dann auf dem ersten Träger immobilisiert werden. Vorteilhafterweise bleibt die Lebensfähigkeit der Mikroorganismen zu einem hohen Prozentsatz so erhalten. Durch Co-Immobilisierung von Mikroorganismen mit entsprechenden Enzymen können unter anderem für den Organismus unvergärbare Substrate vergärbar gemacht werden, und die so entstehenden Produkte mit den Detektionsmolekülen oder der biologischen Probe wechselwirken. Insgesamt kann die Co-Immobilisierung dazu beitragen, dass das Einsatzspektrum des Arrays erweitert wird. Weiterhin können Zellextrakte und Zellkompartimente als Detektionsmoleküle auf dem ersten Träger immobilisiert werden. Viele Molekültypen sind innerhalb der Zelle auf ganz bestimmte Areale - die Kompartimente - begrenzt. Das wird in der Zelle durch Trennung von Membranen, die für bestimmte Stoffe nicht durchlässig sind, erreicht. Ferner wird durch eine Kompartimentierung in den Zellen bzw. Zellextrakten erreicht, dass getrennte Reaktionsräume geschaffen werden, in denen die freie Diffusion weitestgehend unterbunden ist, da die Moleküle oder Enzyme an definierten Strukturen gebunden sind. Vorteilhafterweise können durch das Aufbringen von Zellextrakten oder Zellkompartimenten auch die getrennten Reaktionsräume auf dem ersten Träger immobilisiert werden.

Weiterhin ist es selbstverständlich möglich, auch Liganden als Detektionsmoleküle einzusetzen. Liganden im Sinne der Erfindung sind z.B. Moleküle, wie zum Beispiel Proteine oder Ionen, die um eine Zentralstruktur gruppiert sein können. Liganden können ein und mehrzähnig sein. Unter Liganden können jedoch auch Moleküle verstanden werden, die an spezifische Stellen von Makromolekülen gebunden werden, beispielsweise Substrate oder Coenzyme an ein Protein.

Mit Vorteil können auch Antigene und/oder Antikörper als Detektionsmoleküle eingesetzt werden. Antigene im Sinne der Erfindung sind alle Substanzen, die eine Immunantwort auslösen können. Es kann sich um körperfremde, natürliche oder synthetische Makromoleküle, insbesondere Proteine und Polysaccharide, mit einem Molekulargewicht von über 2 Kilo-Dalton handeln sowie um Oberflächenstrukturen von Fremdpartikeln. Ein Antigen gemäß der Erfindung kann aus einem hochmolekularen Teil bestehen, der als Substrat von meist mehreren niedermolekularen Gruppen, z.B. Haptenen, dient, die für die Spezifität der Immunantwort und die Reaktion der Antigene mit den entsprechenden Immunoglobulinen ausschlaggebend sind. Die Antigene können polyvalent und monovalent sein und so mit einer bzw. mit mehreren Antikörperarten wechselwirken.

Es kann jedoch auch vorgesehen sein, dass statt der Antigene Antikörper auf dem ersten Träger immobilisiert werden. Unter Antikörper werden insbesondere Glycoproteine verstanden, die spezifisch mit einem Antigen wechselwirken. Durch die Wechselwirkung kommt es zur Bildung von Antigen-Antikörper-Komplexen. Die Antikörper können beispielsweise verschiedene Gruppen der Immunglobuline sein. Die Antikörper können selbstverständlich als intakte Antikörper oder als verschiedene Fragmente, die beispielsweise mittels Spaltung von verschiedenen Peptidasen erzeugt werden können, immobilisiert werden. Die Antikörper können vor oder während bzw. nach der Immobilisierung auf dem ersten Träger modifiziert werden, zum Beispiel durch Reduktion, Oxidation oder durch eine Oligomerisation.

Vorteilhaft können auch Rezeptoren als Detektionsmoleküle eingesetzt werden. Rezeptoren sind beispielsweise Proteine, die mit einem extrazellulären Signalmolekül, beispielsweise einem Ligand, in Wechselwirkung treten und durch Konformationsänderungen bestimmte Funktionen, insbesondere über sekundäre Botenstoffe, aktivieren oder initiieren. Rezeptoren im Sinne der Erfindung können jedoch auch spezielle Zellen sein, die Reize aufnehmen und die entsprechende Informationen weiterleiten; Beispiele hierfür wären Foto-, Chemo-, Thermo- und Barorezeptoren.

Weiterhin können auch Lektine als Detektionsmoleküle auf dem ersten Träger immobilisiert werden. Lektine gemäß der Erfindung sind beispielsweise zuckerbindende Proteine oder Glycoproteine nicht-immunen Ursprungs, die Zellen agglutinieren und/oder Glycokonjugate ausfällen. Es kann sich jedoch auch um Proteine handeln, die komplexe Saccharide spezifisch binden, diese jedoch nicht agglutinieren bzw. ausfällen. Bei diesen Proteinen handelt es sich z.B. um monowalente Lektine. Die Lektine können aus Pflanzen, Invertebraten, Vertebraten und/oder Mikroorganismen gewonnen werden. Sie können als Detektionsmoleküle eingesetzt werden, um beispielsweise Erythrocyten, Leukämiezellen, Hefen und Bakterienarten zu binden. Vorteilhafterweise ist die Bindung der Lektine mit Substanzen aus der biologischen Probe saccharidspezifisch. Deshalb agglutinieren die immobilisierten Lektine vorteilhafterweise keine Zellen, die nicht die passenden Oberflächensaccharide tragen, das heißt, derartige Zellen könnten mit dem Erreger oder durch Nicht-Bindung ausgeschlossen werden. Beispiele für einzusetzende Lektine sind beispielsweise das Concanvalin-A aus der Jack-Bohne, die Agglutinine aus Weizenkeimlingen, der Lima-, Garten- und Sojabohne, Rizinus und der Kartoffel. Bei dem Einsatz von Concavalin-A können insbesondere verschiedene Proteinreifungstypen detektiert werden.

Als Detektionsmoleküle können auch sogenannte Aptamere zum Einsatz kommen. Darunter werden insbesondere Peptide, DNA- oder RNA-Moleküle verstanden, die aufgrund ihrer speziellen Molekülform und Ladung andere Moleküle spezifisch binden können.

Nach einer weiteren vorteilhaften Ausführungsvariante der Erfindung werden die Detektionsmoleküle in mehreren Linien auf dem Träger immobilisiert. Beispielsweise kann der erste Träger ein Gel sein, mit dem ein Extrakt oder ein Gemisch aus Detektionsmolekülen aufgetrennt wird. Je nach Auftragsform auf das Gel würden sich so separate Banden oder durchgehende Linien entsprechend des isoelektrischen Punktes, des Molekulargewichts oder ähnlichem ausbilden. Es ist jedoch auch möglich, die Detektionsmoleküle aus dem Gel auf eine Membran zu blotten, die dann als der erste Träger fungiert. Dem Fachmann sind weitere. Methoden bekannt, um die Detektionsmoleküle in mehreren Linien auf dem Träger zu immobilisieren. Mögliche Verfahren wären beispielsweise das Contact Tip Printing, das Ring und Pin Printing, die Photoresistant Lithography und das Micro Wet Printing Verfahren.

In einer weiteren vorteilhaften Ausführungsvariante der Erfindung ist vorgesehen, dass die Detektionsmoleküle durch Western- und/oder Southern-Blot aus einem Gel auf den ersten Träger übertragen werden.

In einer weiteren vorteilhaften Ausführungsform der Erfindung ist vorgesehen, dass als erster Träger Träger umfassend Nitrocellulose, Polyvinylidendifluorid (PVDF), Celluloseacetat, Cellulose-Mischester, Polytetrafluorethylen (PTFE), Polyamid, regenerierte Cellulose, Polycarbonat, Polyester, Polysulfon, Polyacrylamid, Agarose, Nylon und/oder Polybren eingesetzt werden. Der erste Träger kann insbesondere so ausgewählt werden, das eine Immobilisierung von sehr vielen Detektionsmolekülen möglich ist, da der eigentliche zum Einsatz kommende Träger der zweite Träger ist. Mit Vorteil kann der erste Träger insbesondere Nitrocellulose umfassen. Nitrocellulose im Sinne der Erfindung sind anorganische Celluloseester. Weiterhin ist es möglich, dass als erster Träger Nylon eingesetzt wird, wobei Nylon im Sinne der Erfindung lineare aliphatische Polyamide sind, die insbesondere einen hohen Schmelzpunkt aufweisen. Zweckmäßigerweise können auch Polyvinylidenfluoride als erster Träger eingesetzt werden. Polyvinylidenfluoride sind thermoplastische, leicht zu verarbeitende Kunststoffe, die vorteilhafterweise eine hohe Beständigkeit gegen Temperatur- und Chemikalieneinwirkung aufweisen Selbstverständlich ist es möglich, Polyvinylidenfluoride mit unterschiedlichem Kristallinitätsgrad zu verwenden, der durch schnelles bzw. langsames Abkühlen, insbesondere während der Herstellung, erreicht werden kann. Vorteilhafterweise zeichnen sich Polyvinylidenfluoride durch eine hohe mechanische Festigkeit, Steifheit und Zähigkeit auch bei tiefen Temperaturen aus. Zweckmäßigerweise kann der erste Träger auch Celluloseacetat umfassen, wobei im Sinne der Erfindung als Celluloseacetat Celluloseester bezeichnet werden, die durch Umsetzung von Linters oder Zellstoff mit Essigsäureanhydrid in Essigsäure oder Methylenchlorid als Lösungsmittel unter Einsatz von starken Säuren als Katalysatoren im diskontinuierlichen Verfahren hergestellt werden. Vorteilhafterweise zeichnen sich Celluloseacetate durch eine hohe Festigkeit, Schlagzähigkeit, Kratzunempfindlichkeit, Lichtdurchlässigkeit und Oberflächenglanz aus und sie können leicht durch Bedrucken, Lackieren, Heißprägen oder Metallisieren oberflächenveredelt werden. Mit Vorteil kann der erste Träger auch Celluloseester bzw. andere Cellulosederivate umfassen. Cellulosederivate im Sinne der Erfindung sind Substanzen, die durch polymeranaloge Reaktionen chemisch modifizierter Cellulose entstanden sind. Sie umfassen sowohl Produkte, bei denen ausschließlich über Veresterung und/oder Veretherungsreaktionen Hydroxy-Wasserstoffatome der Anhydroglucose-Einheiten der Cellulose durch organische oder anorganische Gruppen substituiert sind, als auch solche, die unter formalem Austausch von Hydroxy-Gruppen der natürlichen Polymeren gegen funktionelle Gruppen, die nicht über ein Sauerstoffatom gebunden sind bzw. über intramolekulare Wasserabspaltung oder Oxidationsreaktionen gebildet werden. Celluloseester im Sinne der Erfindung sind Cellulosederivate, die durch Veresterung von Linters oder Zellstoff mit organischen und/oder anorganischen Säuren bzw. Säurederivaten, die auch als Gemisch eingesetzt werden können, herstellbar sind. Insbesondere Cellulosemischester können gute wasserunlösliche und/oder thermoplastische Eigenschaften aufweisen. Selbstverständlich ist es möglich, dass der erste Träger auch andere Kunststoffe und/oder Polymerisate umfasst. Zu den Kunststoffen im Sinne der Erfindung gehören abgewandelte Naturstoffe, wie Duroplaste und Thermoplaste ebenso wie synthetische Kunststoffe, beispielsweise Polykondensate, Polymerisate und Polyaddukte. Beispiele für derartige Verbindungen sind: Acrylnitril-Butadien-Styrol, Acrylnitril-Methylmethacrylat, Celluloseacetobutyrat, Kresol-Formaldehyd, Carboxymethylcellulose, Casein, Diallylphthalat, Ethylcellulose, Epoxid, expandierbares Polystyrol, Ethylen-Vinylacetat, Ethylen-Vinylalkohol, Tetrafluorethylen-Hexafluorpropylen, Polyethylen hoher Dichte (Hart-PE), Polyethylen niederer Dichte (Weich-PE), Methylmethacrylat-Butadien-Styrol, Methylcellulose, MelaminFormaldehyd, Polyamid, Polymeres aus γ-Carprolactam, Polykondensat aus Hexamethylendiamin u. Adipinsäure, Polyacrylnitril, Polybuten, Polybutylenterephthalat, Polycarbonat, Polychlortrifluorethylen, Polyethylen, chloriertes Polyethylen, Ethylen-Propylen, Polyethylenterephthalat, Phenol-Formaldehyd, Polyimid, Polyisobutylen, Polymethylmethacrylat, Polyoxymethylen, Polyacetal, Polypropylen, Polyphenylenoxid, Polyphenylensulfid, Polystyrol, Polytetrafluorethylen, Polyurethan, Polyvinylacetat, Polyvinylalkohol, Polyvinylchlorid, Polyvinylcarbazol, Polyvinylpyrrolidon, Styrol-Acrylnitril, Polystyrol mit Elastomer auf der Basis von Butadien modifiziert, Silicon, Styrol - α-Methylstyrol, Harnstoff-Formaldehyd und/oder ungesättigte Polyester.

Vorteilhafterweise weisen derartige Kunststoffe ein niedriges spezifisches Gewicht, eine hohe Beständigkeit gegen Korrosion sowie eine hohe elektrische Isolierfähigkeit, leichte Formgebung und eine gute Bedruckbarkeit, insbesondere für Detektionsmoleküle, auf. Polymerisate im Sinne der Erfindung sind Produkte, die aus Polyreaktionen, beispielsweise Polymerisation, Polyaddition und/oder Polykondensation bzw. polymeranalogen Reaktionen hervorgegangen sind. Monomere, die zu polymeren Verbindungen führen, können beispielsweise Ethylen, Styrol, Vinylchlorid, Vinylacetat, Methylmethacrylat und/oder Ethylenoxid sein.

In einer weiteren vorteilhaften Ausführungsvariante der Erfindung ist vorgesehen, dass als aushärtendes Material gesättigte, aliphatische Kohlenwasserstoffe, insbesondere Paraffin, Einbettungsharze, Collagen-Lösungen, wässrige Lösungen, Lösungen sich vernetzender Proteine, Kohlenhydrate, Nukleinsäuren, Polymere und/oder Agarose eingesetzt werden. Kohlenwasserstoffe im Sinne der Erfindung sind organische Verbindungen, die Kohlenwasserstoff und Wasserstoff umfassen. Es ist selbstverständlich auch möglich, dass die Kohlenwasserstoffe weitere Substanzen oder Verbindungen wie beispielsweise Sauerstoff, Stickstoff, Schwefel, Phosphor bzw. spezifische funktionelle Gruppen umfassen. Paraffine im Sinne der Erfindung sind ein festes oder flüssiges Gemisch aus insbesondere gesättigten aliphatischen Kohlenwaserstoffen, das vorteilhafterweise farb-, geruch- und geschmacklos und sich in Xylol, Äther und Chloroform leicht, in Wasser und 70%igem Alkohol nicht löst und das nicht fluoresziert. Collagenlösungen im Sinne der Erfindung umfassen Collagen. Collagene sind langfaserige, linearkolloide, hochmolekulare Skleroproteine, die insbesondere in der extrazellulären Matrix, in Bindegeweben, in der eiweißhaltigen Grundsubstanz des Knochens und im Dentin vorkommen. Agarose im Sinne der Erfindung sind gelierfähige Polyasaccharide aus Agar, das insbesondere aus wechselnden Einheiten von β-1,3-verknüpfter D-Galaktopyranose und α-1,4-verknüpfter 3,6-Anhydro-L-galaktopyranose besteht, wobei die Galaktose in 6-Stellung methyliert sein kann. Wässrige Lösungen sind Lösungen, die Wasser umfassen und die insbesondere bei niedrigen Temperaturen aushärten.

In einer weiteren vorteilhaften Ausführungsvariante der Erfindung ist vorgesehen, dass als zweiter Träger Träger umfassend Metall, Polypropylen, Teflon, Polyethylen, Polyester, Polystyren, Keramik und/oder Glas eingesetzt werden. Metalle im Sinne der Erfindung sind alle Verbindungen, deren Zusammenhalt durch ein Kristallgitter entsteht. Die Grenze zwischen Metallen und Nichtmetallen ist fließend, so dass auch die Elemente Ce, Sn, As und Sb im Sinne der Erfindung Metalle sind. Zu den Metallen gemäß der Erfindung gehören auch die metallischen Gläser, das heißt Werkstoffe, die sich in einem metastabilen, weitgehend amorphen Zustand befinden. Selbstverständlich sind auch metallisch leitfähige Polymere Metalle im Sinne der Erfindung. Vorteilhafterweise weisen Metalle im Sinne der Erfindung insbesondere eine gute Festigkeit, eine gute Härte und Verschleißbeständigkeit, eine hohe Zähigkeit und eine gute elektrische und thermische Leitfähigkeit auf. Polypropylene im Sinne der Erfindung sind thermoplastische Polymere des Propylens. Polypropylene zeichnen sich insbesondere durch eine hohe Härte, Rückstellfähigkeit, Steifheit und Wärmebeständigkeit aus. Der zweite Träger kann jedoch auch Teflon umfassen. Teflon gemäß der Erfindung sind Polytetrafluoroethylene, die vorteilhafterweise gute thermoplastische Eigenschaften aufweisen. Polyethylene entstehen insbesondere durch eine Polymerisation von Ethylen nach im wesentlichen zwei unterschiedlichen Methoden, dem Hochdruck- und dem Niederdruckverfahren. Polyethylene, die im Hochdruckverfahren hergestellt werden, weisen vorteilhafterweise eine geringe Dichte auf. Die Eigenschaften von zweiten Trägern, die Polypropylen umfassen, werden im wesentlichen durch den Charakter des Polyethylen als partiell kristallinem Kohlenwasserstoff bestimmt. Vorteilhafterweise sind Polyethylene bis zu 60° Grad in allen üblichen Lösungsmitteln praktisch unlöslich. Vorteilhafterweise bewirken polare Flüssigkeiten wie Alkohol, Ester und Ketone bei Zimmertemperatur kaum eine Quellang von Polyethylenen. Gegen Wasser, Laugen und Salzlösungen sowie anorganischen Säuren verhalten sich Polyethylene vorteilhafterweise völlig indifferent. Träger, die Polyethylene umfassen, haben z.B. eine sehr geringe Wasserdampfdurchlässigkeit. Der zweite Träger kann jedoch zweckmäßigerweise auch Polyester umfassen. Polyester im Sinne der Erfindung sind Verbindungen, die durch Ringöffnungspolymerisation von Lactonen oder durch Polykondensation von Hydroxycarbonsäuren bzw. von Diolen und Dicarbonsäuren bzw. Dicarbonsäurederivaten hergestellt werden. Polyester im Sinne der Erfindung umfassen auch Polyesterharze, Polyesterimide, Polyesterkautschuke, Polyesterpolyole und Polyesterpolyuretane. Polyester sind vorteilhafterweise Thermoplaste und besitzen ausgesprochenen Werkstoffcharakter. Sie zeichnen sich beispielsweise durch eine hohe Thermostabilität aus und können zu Legierungen mit Metallen, wie beispielsweise Kupfer, Aluminium und Magnesium, verarbeitet werden. Es kann jedoch auch vorgesehen sein, dass der zweite Träger Keramik umfaßt. Keramik im Sinne der Erfindung ist eine Sammelbezeichnung für insbesondere anorganische und überwiegend nicht-metallische Verbindungen und Elementen aufgebaute und insbesondere zu mehr als 30 Vol.% kristalline Materialien umfassende Stoffklasse. Dem Fachmann sind verschiedene Keramiken bzw. keramische Werkstoffe bekannt, die er als zweiten Träger einsetzen kann. Es kann sich beispielsweise um sogenannte Töpferware, Steingutgeschirr, Spaltplatten, Laborporzellan, Geschirrporzellan, Knochenporzellan, Aluminiumoxidkeramik, Dauermagnetwerkstoffe, Silicasteine und Magnesiasteine handeln. Bei tonkeramischen Werkstoffen wird im Sinne der Erfindung in grobe und feine Werkstoffe unterschieden, wobei feine tonkeramische Werkstoffe Irdengut, Steingut, Steinzeug und Porzellan umfassen. Es können jedoch mit Vorteil auch sonderkeramische Werkstoffe wie Glas-, Oxidkeramik, SiC-Steine und schmelzflüssig gegossene Steine als zweite Träger eingesetzt werden. Bevorzugt kann der zweite Träger auch Glas umfassen. Glas im Sinne der Erfindung sind Stoffe im amorphen, nichtkristallinen Festzustand, das heißt, der Glaszustand lässt sich im Sinne der Erfindung als eingefrorene unterkühlte Flüssigkeit bzw. Schmelze auffassen. Gläser sind daher anorganische oder organische, meist oxidische Schmelzprodukte, die durch einen Einführvorgang ohne Auskristallisation der Schmelzphasenkomponenten in einen festen Zustand überführt wurden. Selbstverständlich sind im Sinne der Erfindung auch Kristalle, Schmelzen und unterkühlte Schmelzen als Gläser aufzufassen. Die Gläser können zum Beispiel Flachglas, Behälterglas, Wirtschaftsglas, Laborgeräteglas, Bleikristallglas, Faserglas, optische Glasfasern und andere sein. Selbstverständlich ist es auch möglich, dass Silicat-freie Gläser, beispielsweise Phosphatgläser eingesetzt werden. Der zweite Träger kann jedoch auch so beschaffen sein, dass optische Gläser, das heißt, z.B. Gläser mit besonderen optischen Brechungsindexen eingesetzt werden.

In einer weiteren vorteilhaften Ausführungsvariante der Erfindung ist vorgesehen, dass die Oberfläche der Träger modifiziert wird. Die Modifizierung der Träger kann insbesondere durch Einwirken von biologischen, physikalischen und/oder chemischen Einflüssen erfolgen. Physikalisches Einwirken wäre beispielsweise das Polieren, Ätzen, Beizen, Sandstrahlen, aber auch physikalische Verfahren, die zu einem Härten, Beschichten, Vergüten, Überziehen mit Schutzhäuten und ähnlichem führen. Eine Oberflächenbehandlung durch biologische Einwirkung kann beispielsweise das Bewachsen durch Mikroorganismen umfassen. Eine chemische Modifikation der Oberfläche der Träger beinhaltet beispielsweise die Behandlung mit Säuren, Basen, Metalloxiden und anderem. Die Oberfläche der Träger kann so modifiziert werden, dass die Detektionsmoleküle auf dem Träger besonders gut haften bzw. so haften, dass sie in ihrer Aktivität nicht nachteilig modifiziert werden. Die Oberflächenmodifizierung umfasst auch das Beschichten mit Poly-L-Lysinen, Aminosilanen, Aldehydsilanen, EpoxyGruppen, Gold, Streptavidin, verzweigte Linker, reaktive Gruppen, Polyacrylamid-Pads, immobilisierte Nitrocellulose und/oder aktivierten Aldehyden bzw. Agarose-AldehydGruppen, wodurch insbesondere gebunden werden: DNA, COO-re gebunden werden: DNA, COO⁻-Gruppen, NH₂-Gruppen, Biotin, Thiol-Gruppen und andere. Eine Oberflächenmodifizierung der Träger umfasst selbstverständlich auch eine Behandlung, die zu einer erhöhten Stabilität und Bruchfestigkeit insbesondere des zweiten Trägers führt. Es können selbstverständlich auch klassische Oberflächenmodifizierungen aus der Histologie vorgenommen werden, wie das Beschichten des ersten oder zweiten Trägers mit z.B. Eiweißglycerin, Polylysin, aktivierten Dextranen oder Chromgelatine. Ebenso ist das Aufschmelzen oder Antrocknen des ersten Trägers auf dem zweiten Träger möglich. Antrocknen ist ein Vorgang, der bevorzugt zwischen 20 und 100°C ablaufen kann und in einer besonders bevorzugten Ausführungsvariante zwischen 37 °C und 80 °C vorgenommen werden kann.

In einer weiteren vorteilhaften Ausführungsform der Erfindung ist vorgesehen, dass als Lösungsmittel, vor allem zum teilweisen oder vollständigen Auf- oder Ablösen des Einbettungsmediums oder des ersten Trägers, Lösungsmittel umfassend Chloroform, Methanol, Ethanol, Amylacetat, Amylalkohol, Cyclohexanon, Dimethylsulfoxid, Diethylacetamid, Dimethylformamid, Aceton, Acetonitril, Isopropylacetat, Methylenchlorid, Methylketon, Methylisobutylketon, Cellosolve, Tetrahydrofuran, Methylacetat, Pyridin, Butylacetat, Dioxan, Isopropylacetat, Ethylacetat, Dimethylacetamid, Trifluoressigsäure, Oxidationsmittel, Säuren, Basen und/oder Enzyme eingesetzt werden. Lösungsmittel im Sinne der Erfindung sind Stoffe, die andere auf biologischem, chemischem und/oder physikalischem Wege zur Lösung bringen können, insbesondere aber anorganische und organische Flüssigkeiten, die andere gasförmige, flüssige oder feste Stoffe zu lösen vermögen. Bei den anorganischen Lösungsmitteln kann beispielsweise in Protonen-haltige und Protonen-freie Lösungsmittel sowie in wässrige und nicht-wässrige Lösungsmittel unterschieden werden. Organische Lösungsmittel sind beispielsweise Alkohole - wie Methanol, Ethanol, Propanole, Butanole, Oktanole und Cyclohexanol-, Glykole - wie beispielsweise Ethylenglykol- und Diethylglykol-, Ether und Glykolether, - wie beispielsweise Diethylehter, Dibutylether, Anisol, Dioxan, Tetrahydrofuran, Mono-, Di-, Tri-, und/oder Polyethylenglykolether-, Ketone - wie beispielsweise Aceton, Butanon und Cyclohexanon-, Ester - wie beispielsweise Essigsäureester und Glykolester -, Amide und andere Stickstoffverbindungen - wie beispielsweise Dimethylformamid, Pyridin und Acetonitril-, Schwefelverbindungen - wie beispielsweise Schwefelkohlenstoff, Dimethylsulfoxid und Sulfolan-, Nitroverbindungen - wie beispielsweise Nitrobenzol-, Halogenkohlenwasserstoffe - wie beispielsweise Dichlormethan, Chloroform, Tetrachlormethan, Tri-, Tetracholrethen, Ethylenchlorid und Chlorfluorkohlenwasserstoffe-, Kohlenwasserstoffe - wie beispielsweise Benzine, Petrolether, Cyclohexan, Methylcyclohexan, Dekalin, Terpen-Lösungsmittel, Benzol, Toluol, Xylole und Propylenoxid. Als Lösungsmittel können jedoch auch Oxidationsmittel eingesetzt werden. Beispielsweise solche Mittel, die leicht Sauerstoff abgeben und somit auf andere übertragen können, wie beispielsweise Kaliumpermanganat, Kaliumchlorat und/oder Blei(IV)-oxid sowie auch solche, die dehydrierend wirken, also anderen Wasserstoff entziehen oder Wasserstoff aufnehmen können, wie beispielsweise Jod und Photooxidantien; das heißt erfindungsgemäß sind Oxidantien allgemein solche Elemente und Verbindungen, die bestrebt sind, durch Aufnahme von Elektronen unter Übergang zu einem energieärmeren Zustand stabile Elektronenschalen auszubilden, zum Beispiel Natriumethanolat, Natrium-Meta-Perjodat und Wasserstoff-peroxid. Selbstverständlich kann das Lösungsmittel auch Enzyme umfassen. Beispiele für derartige Enzyme sind: Oxidoreduktasen, Transferasen, Hydrolasen, Lyasen, Isomerasen und/oder Ligasen, Proteasen, Kollagenase. Enzyme im Sinne der Erfindung sind alle Proteine, Peptide, Lipide und/oder Kohlenhydrate, die als Biokatalysatoren chemische Reaktionen modifizieren können, zum Beispiel Cellulase, Gelatinase oder Agarase.

In einer weiteren vorteilhaften Ausführungsform der Erfindung ist vorgesehen, dass zum Separieren der eingebetteten Träger ein Mikrotom, ein Cryomikrotom, ein Ultramikrotom, ein Ultracryomikrotom und/oder ein Vibratom eingesetzt werden kann. Ein Mikrotom zum Anfertigen von Schnitten ist besonders dann geeignet, wenn als Einbettungsmedium Paraffin eingesetzt wird. Ein Cryomikrotom zeichnet sich z.B. dadurch aus, dass die Proben durch die Bearbeitung bei tiefen Temperaturen von minus 10 bis minus 70 °C besonders schonend behandelt werden und bevorzugt wässrige Einbettungsmedien mit dem gleichen schonenden Effekt verwendet werden können. Ein Ultramikrotom wird dann eingesetzt, wenn als Einbettungsmedium Kunstharze eingesetzt werden und/oder besonders dünne Schnitte mit einer Dicke von insbesondere 20 nm bis 1 µm hergestellt werden. Derartig dünne Schnitte bewirken einen besonders sparsamen Materialverbrauch der auf dem ersten Träger immobilisierten Materialien. Die Ultramikrotomie kann selbstverständlich auch als Cryomikrotomie ausgeführt werden. Ein Vibratom wird besonders dann eingesetzt, wenn der zu schneidende erste Träger eine Konsistenz aufweist, die vergleichbar mit chemisch fixiertem, biologischem Gewebe ist. In diesem Falle muß nämlich kein zusätzliches Einbettungsmedium.zum Anfertigen der Schnitte eingesetzt werden. Das Vibratom kann auch eingesetzt werden, wenn das Einbettungsmedium die entsprechende Konsistenz aufweist, wie z.B. mit Glutardialdehyd vernetztes Serumalbumin und/oder ausgehärtetes Kollagen.

In einer weiteren vorteilhaften Ausführungsvariante der Erfindung ist vorgesehen, dass auf dem ersten Träger immobilisierte Detektionsmoleküle durch Inkubation mit Formaldehyd, Glutaraldehyd, Eisessig, bivalente Kopplungsreagenzien und/oder durch polyvalente Kopplungsreagenzien, insbesondere oxydiertes Dextran, tosylaktiviertes Dextran, Polylysin, aktiviertes Polylysin, Proteine, aktivierte Proteine, aktivierte Nukleinsäuren, aktivierte Polycarbonate und/oder Polyethylenglykole vor und/oder während und/oder nach der Ablösung des ersten Trägers miteinander vernetzt werden. Durch die Inkubation mit den verschiedenen Substanzen werden die Detektionsmoleküle so vernetzt, dass sie gut auf den zweiten Träger aufgebracht werden können.

Ein Vorteil des erfindungsgemäßen Verfahrens ist es, dass durch die Trennung des Prozesses der Immobilisierung auf den ersten Träger und das Aufbringen von Schnitten auf einen zweiten Träger die Träger spezifischer und effizienter ausgewählt werden können, als dies der Fall wäre, wenn der Träger auf dem die Detektionsmoleküle immobilisiert werden auch der zur Detektion verwendete Träger ist. Der erste Träger wird vor allem so ausgewählt, dass möglichst viele Detektionsmoleküle immobilisiert werden können, beispielsweise durch vorhandene hydrophobe oder hydrophile Wechselwirkungen, durch spezielle funktionelle Gruppen, durch Epoxy-Aktivierung, Aldehyd-Aktivierung, verzweigte Linker oder durch elektrisch geladene makromolekulare aufgebrachte Materialien. Der zweite Träger hingegen wird speziell nach Gesichtspunkten des jeweiligen Einsatzes des Arrays konzipiert. Für den Einsatz in der klinischen Diagnostik haben sich beispielsweise Glasarrays oder Teststreifen aus Cellulose gut etabliert, auf die bisher jedoch die Detektionsmoleküle nicht in der erforderlichen Dichte, Reproduzierbarkeit und Effizienz aufgebracht und immobilisiert werden konnten.

Ein weiterer Vorteil des Verfahrens ist, dass die Detektions- und/oder Biomoleküle, die auf den ersten Träger aufgebracht werden, untereinander vernetzt werden können und nach Auflösen des ersten Trägers ein dreidimensionales Netzwerk auf dem zweiten Träger bilden können, das optimal für Komponente und/oder Probenmoleküle zugänglich ist.

Die Verwendung von zwei Trägern ermöglicht es, z.B. einen ersten Träger mit hoher Bindungskapazität für Detektionsmoleküle und einen zweiten Träger mit guten optischen Eigenschaften, d.h. beispielsweise mit geringer Eigenfluoreszenz, zu verwenden. Durch vollständiges oder teilweises Auflösen des ersten Trägers können störende optische Eigenschaften des ersten Trägers verringert oder vermieden werden. Es gelingt aber gleichzeitig, eine hohe Beladungsdichte von Detektionsmolekülen auf dem zweiten Träger sicherzustellen, der eine optimale Fluoreszenzdetektion ermöglicht.

Ein weiterer Vorteil des erfindungsgemäßen Verfahrens ist, dass die Detektionsmoleküle sehr dicht gepackt immobilisiert werden können. Die Packungsdichte ergibt sich insbesondere aus der Spot- und/oder Liniendichte der Detektionsmoleküle auf einem ersten Träger und der Packung von mehreren ersten Trägern übereinander. Die Arrays sind mit dem erfindungsgemäßen Verfahren mit hoher Reproduzierbarkeit und kostengünstig herstellbar. Durch Übertragung des ersten Trägers auf den Boden einer Mikrotestplatte wird das Detektionssytem in eine automatisierbare Systemumgebung gebracht, die in vielen Labors und insbesondere in Routinelabors zum Standard gehören.

Die Erfindung soll im folgenden anhand eines Beispiels näher erläutert werden, ohne dass die Erfindung auf dieses Beispiel zu beschränken ist.

### Beispiel 1

### Übertragung eines Westernblots von Nitrocellulose auf einen Glasobjektträger und anschließende Bestimmung von pathögenspezifischem Human-IgG.

Bakterielle Rohextrakte von Borrelia burgdorferi, Chlamydia trachomatis, Yersinia enterocolytica, Campylobacter jejuni, Mycoplasma pneumoniae und Salmonella spec. werden durch Verdünnung mit destilliertem Wasser auf eine Proteinkonzentration von 1 mg/ml eingestellt. Anschließend werden 10 *µ*g eines jeden Rohextraktes in einer Polyacrylamidgel-Disk-Elektrophorese (Laemmli [1970], Nature, 227; 680-685) aufgetrennt (10%-Gel, 1mm Gelstärke, 10er Kamm, Elektrophoresekammer Mini-Protean II [Biorad]). Mittels Elekttroblotting (Towbin et al.[1979] Proc. Natl. Acad. Sci. U.S.A. 76, 4350-4354) werden die aufgetrennten Proteine aus dem Gel auf eine Nitrocellulosemembran (Life Technologies) übertragen.

Die getrockneten Bahnen jedes bakteriellen Rohextraktes werden aus der Nitrocellulosefolie ausgeschnitten und in 20 Stücke von 0,5 cm zerschnitten. Die Stücke werden in definierter Reihenfolge übereinander in einen biegsamen Metallrahmen gelegt, der an einer Stelle unterbrochen ist und sich in einer Glasschale mit planarem Boden und einem Durchmesser von 5 cm befindet. Zwischen die Nitrocellulosestücke, die geblottete Proteine tragen, werden als Abstandhalter 3 Nitrocellulosestücke identischer Größe gelegt. Die Glasschale wird auf 60° Celsius vorgewärmt und bis zu einer Füllhöhe von 1 cm mit flüssigem Paraffin gefüllt, ohne dass die Reihenfolge der Nitrocellulosestücke verändert wird. Es ist darauf zu achten, dass sich eventuell zwischen den Nitrocellulosestücken befindliche Luftblasen durch vorsichtiges Bewegen oder durch Anlegen eines Vakuums entfernt werden. Die mit Paraffin gefüllte Schale wird über Nacht bei 60° Celsius inkubiert. Danach wird die Schale zügig abgekühlt und der Metallrahmen mit den Nitrocellulosestreifen aus dem Gesamtblock herausgeschnitten. Durch Aufbiegen des Metallrahmens wird letztlich der Block mit den Nitrocellulosestücken freigelegt und auf ein Mikrotom zum Anfertigen der Paraffinschnitte befestigt.

Es werden je ein Schnitt von einer Dicke von 10 *µ*m angefertigt und auf Glasobjektträger aufgebracht, die nach Standardprozeduren mit Eiweißglycerin behandelt wurden. Nach dem "Strecken" des Paraffinschnittes auf einem Wassertropfen bei 60°Celsius wird das Wasser abgesaugt, der Schnitt positioniert und bei 37°Celsius für ca. 2 h angetrocknet.

Nach dem Trocknen der Schnitte werden sie durch Einstellen des Objektträgers in Xylol entparaffiniert. Danach werden die Objektträger zweimal in 100% Ethanol und zweimal in 100% Aceton für je 5 Minuten gewaschen und anschließend in Wasser für je 2 Minuten rehydratisiert und in 0,05 M Natrium-Phosphat-Puffer, pH 7,4 + 0,1% Tween für 15 Minuten equilibriert.
Anschließend werden die Objektträger in 1% humanem Serumalbumin in PBS-T für 30 Minuten geblockt.

Die Objektträger werden nun mit humanen Seren, die 1:100 in PBS-T verdünnt wurden, für 1 h bei Raumtemperatur inkubiert. Danach werden die Objektträger in PBS-T eingestellt und unter dreimaligem Wechseln der Spülflüssigkeit für 30 Minuten gewaschen. Anschließend erfolgt die Inkubation mit ScreenBeads-Rhodamin (Chemicell), an die Anti-human-IgG-Antiserum (IgG-Fraktion) (Rockland) gekoppelt wurde. In einer Verdünnung von 1:1000 in PBS-T für 1 h bei Raumtemperatur. Nach dem Spülen der Objektträger mit PBS-T unter dreimaligem Wechseln der Spülflüssigkeit für 30 Minuten und kurzem Eintauchen in destilliertes Wasser werden die Objektträger getrocknet und mit dem Laserfluoskop (IOM) ausgewertet. Die gemessene Rhodaminfluoreszenz ist proportional zur Menge des spezifisch gebundenen humanen IgG. Alternativ kann an das Anti-human-IgG-Antiserum (IgG-Fraktion) eine Peroxidase gekoppelt sein. Nach der Inkubation mit dem Antikörper-Peroxidasekonjugat und dem sich anschließenden Spülschritt werden die Objektträger mit Diaminobendzidin-Substratlösung nach Standardverfahren inkubiert. Die Auswertung erfolgt in diesem Falle mit einem Scanner oder einer CCD-Kamera.

### Beispiel 2

### Übertragung eines Western-Blots von Nitrozellulose auf eine PVDF-Membran und Bestimmung von pathogenspezifischem Human IgG

Ein bakterieller Rohextrakt von Borrelia burgdorferi wird entsprechend Beispiel 1 in der Polyacrylamidgelelektrophorese aufgetrennt und auf eine Nitrozellulosemembran geblottet. Aus der Nitrozelluloseblotmembran werden folgende spezifische Banden mit einem Skalpel ausgeschnitten: 83 kD, 41 kD, 39 kD, 34 kD, 31 kD (Osp A), 28/29 kD (Osp D), 25 kD (Osp C), 21 kD. Dabei können die Banden aus verschiedenen Blots zusammengestellt werden, um eine optimale Repräsentation des jeweiligen Proteins zu gewährleisten. Die ausgeschnittenen Banden werden dann im Abstand von 2 mm auf eine zweite Membran (Nitrozellulose oder PVDF) in definierter Anordnung aufgeklebt. Die zweite Membran wird nun in einen Paraffinblock eingeschlossen und senkrecht der Längsausdehnung der Proteinbanden in 10 *µ*m dicke Schnitte geschnitten. Die Schnitte werden bei 56 °C auf einer Wasseroberfläche gestreckt und dann auf eine PVDF-Membran übertragen und bei 45 °C getrocknet. Nachfolgend wird das Paraffin durch Inkubation mit Xylol oder Rotihistol (Roth) vollständig entfernt. Dazu wird die PVDF-Membran auf ein mit dem Lösungsmittel getränktes Filterpapier übertragen und zwei Stunden in einer geschlossenen Kammer inkubiert. Danach wird die PVDF-Membran für zweimal 5 min in gleicher Weise mit 100 % Methanol und anschließend für 1 h in DMSO und dann für eine Stunde mit Wasser inkubiert. Danach wird die PVDF-Membran in 0,05 M Tris-HCl-Puffer, pH 7,4 und 0,1 % Tween 20 (TBS-T) equilibriert und anschließend durch Zugabe von 1 % humanem Serumalbumin für eine Stunde geblockt.

Anschließend werden die so gefertigten Membranen mit Patientenseren, die zuvor in TBS-T verdünnt worden sind, eine Stunde inkubiert. Nach mehrmaligem Waschen in TBS-T wurden die Membranen mit Anti-human-IgG-Peroxidasekonjugat (Rockland) in einer Verdünnung von 1:2000 in TBS-T für eine Stunde inkubiert. Nach mehrmaligem Waschen in TBS-T erfolgte die Peroxidasesubstratreaktion unter Verwendung von TMB-Fertiglösung (Seramun) für 15 min.
Alle Inkubationen des Immunnachweises erfolgten bei Raumtemperatur.

## Patentansprüche

1. Verfahren zur Herstellung eines Arrays zur Detektion von Komponenten aus einer biologischen Probe,
**dadurch gekennzeichnet, dass**
- Detektionsmoleküle die durch Transferverfahren ausgewählt aus der Gruppe umfassend Elektroblotting, Kapillarblotting, Auf sprühen oder Aufdrucken auf einem oder mehreren ersten Trägern immobilisiert werden, wobei der erste Träger eine Membran umfasst,
- der oder die erste(n) Träger in einem Material eingebettet wird/werden,
- das Material zum Aushärten gebracht wird,
- der/die eingebettete (n), erste (n) Träger senkrecht in Schnitte separiert wird/werden;
- mindestens ein Schnitt auf einen zweiten Träger aufgebracht wird,
- von dem aufgebrachten Schnitt mit einem Lösungsmittel das ausgehärtete Material und/oder der/die erste(n) Träger vollständig oder teilweise auf- oder abgelöst werden und der Array erhalten wird.

2. Verfahren nach Anspruch 1,
**dadurch gekennzeichnet, dass**
mindestens zwei erste Träger überlappend in dem Material eingebettet werden.

3. Verfahren nach Anspruch 1 oder 2,
**dadurch gekennzeichnet, dass**
als Detektionsmoleküle Mikroorganismen, Zellextrakte, Liganden, Antigene, Antikörper, Rezeptoren, Nukleinsäuren, Lektine, Proteine, Peptide, Glycopeptide, Kohlenhydrate, Nukleinsäuren und/oder Lipide eingesetzt werden.

4. Verfahren nach einem der vorhergehenden Ansprüche,
**dadurch gekennzeichnet, dass**
die Detektionsmoleküle in mehreren Linien auf dem ersten Träger immobilisiert werden.

5. Verfahren nach einem der vorhergehenden Ansprüche,
**dadurch gekennzeichnet, dass**
die Detektionsmoleküle durch Western- und/oder Southern-Blot aus einem Gel auf den ersten Träger übertragen werden.

6. Verfahren nach einem der vorhergehenden Ansprüche,
**dadurch gekennzeichnet, dass**
als erste Träger Träger umfassend Nitrocellulose, Polyvinylidendifluorid (PVDF), Celluloseacetat, Cellulose-Mischester, Polytetrafluorethylen (PTFE), Polyamid, regenerierte Cellulose, Polycarbonat, Polyester, Polysulfon, Polyacrylamid, Agarose, Nylon und/oder Polybren eingesetzt werden.

7. Verfahren nach einem der vorhergehenden Ansprüche,
**dadurch gekennzeichnet, dass**
als aushärtendes Material gesättigte, aliphatische Kohlenwasserstoffe, insbesondere Paraffin, Einbettungsharze, Collagenlösungen, wässrige Lösungen, Lösungen sich vernetzender Proteine, Kohlenhydrate, Nukleinsäuren, Polymere und/oder Agarose eingesetzt werden.

8. Verfahren nach einem der vorhergehenden Ansprüche,
**dadurch gekennzeichnet, dass**
als zweite Träger Träger umfassend Metall, Polypropylen, Teflon, Polyethylen, Polystyren, Polyester, Keramik und/oder Glas eingesetzt werden.

9. Verfahren nach einem der vorhergehenden Ansprüche,
**dadurch gekennzeichnet, dass**
die Oberfläche der Träger modifiziert wird.

10. Verfahren nach einem der vorhergehenden Ansprüche,
**dadurch gekennzeichnet, dass**
als das Lösungsmittel Lösungsmittel umfassend Chloroform, Methanol, Ethanol, Amylacetat, Amylalkohol, Cyclohexanon, Dimethylsulfoxid, Diethylacetamid, Dimethylformamid, Aceton, Acetonitril, Isopropylacetat, Methylenchlorid, Methylketon, Methylisobutylketon, Cellosolve, Tetrahydrofuran, Methylacetat, Pyridin, Butylacetat, Dioxan, Isopropylacetat, Ethylacetat, Dimethylacetamid, Trifluoressigsäure, Oxidationsmittel, Säuren, Basen und/oder Enzyme, eingesetzt werden.

11. Verfahren nach einem der vorhergehenden Ansprüche,
**dadurch gekennzeichnet, dass**
zum Separieren der eingebetteten Träger ein Mikrotom, ein Cryomikrotom, ein Ultramikrotom, ein Ultracryomikrotom und/oder ein Vibratom eingesetzt werden.

12. Verfahren nach einem der vorhergehenden Ansprüche,
**dadurch gekennzeichnet, dass**
auf dem ersten Träger immobilisierte Detektionsmoleküle durch Inkubation mit Formaldehyd, Glutaraldehyd, Eisessig, bivalenten Kopplungsreagenzien und/oder durch polyvalente Kopplungsreagenzien, insbesondere oxydiertes Dextran, tosylaktiviertes Dextran, Polylysin, Proteine, aktivierte Proteine, aktiviertes Polylysin, aktivierte Polycarbonate und/oder Polyethylenglykole vor und/oder während und/oder nach der Ablösung des ersten Trägers miteinander vernetzt werden.

## Claims

1. A method of producing an array for the detection of components from a biological sample,
**characterized in that**
- detection molecules are immobilized on one or more first supports using transfer procedures selected from the group comprising electroblotting, capillary blotting, spraying, or imprinting, said first support comprising a membrane,
- the first support(s) is/are embedded in a material,
- said material is caused to cure,
- the embedded first support(s) is/are separated vertically into sections,
- at least one section is applied on a second support,
- the cured material and/or the first support(s) are completely or partially dissolved or removed from the applied section using a solvent, and the array is obtained.

2. The method according to claim 1,
**characterized in that**
at least two first supports are embedded in overlap in the material.

3. The method according to claim 1 or 2,
**characterized in that**
microorganisms, cell extracts, ligands, antigens, antibodies, receptors, nucleic acids, lectins, proteins, peptides, glycopeptides, carbohydrates, and/or lipids are employed as detection molecules.

4. The method according to any of the preceding claims,
**characterized in that**
the detection molecules are immobilized on the first support in the form of multiple lines.

5. The method according to any of the preceding claims,
**characterized in that**
the detection molecules are transferred from a gel onto the first support by means of a Western blot and/or Southern blot.

6. The method according to any of the preceding claims,
**characterized in that**
supports comprising nitrocellulose, polyvinylidene difluoride (PVDF), cellulose acetate, cellulose mixed esters, polytetrafluoroethylene (PTFE), polyamide, regenerated cellulose, polycarbonate, polyester, polysulfone, polyacrylamide, agarose, nylon, and/or polyprene are used as first supports.

7. The method according to any of the preceding claims,
**characterized in that**
saturated aliphatic hydrocarbons, especially paraffin, embedding resins, collagen solutions, aqueous solutions, solutions of crosslinking proteins, carbohydrates, nucleic acids, polymers, and/or agarose are used as curing material.

8. The method according to any of the preceding claims,
**characterized in that**
supports comprising a metal, polypropylene, teflon, polyethylene, polystyrene, polyester, ceramics, and/or glass are used as second supports.

9. The method according to any of the preceding claims,
**characterized in that**
the surfaces of the supports are modified.

10. The method according to any of the preceding claims,
**characterized in that**
solvents comprising chloroform, methanol, ethanol, amyl acetate, amyl alcohol, cyclohexanone, dimethylsulfoxide, diethylacetamide, dimethylformamide, acetone, acetonitrile, isopropyl acetate, methylene chloride, methyl ketone, methyl isobutyl ketone, cellosolve, tetrahydrofuran, methyl acetate, pyridine, butyl acetate, dioxane, ethyl acetate, dimethylacetamide, trifluoroacetic acid, oxidants, acids, bases, and/or enzymes are used as solvents.

11. The method according to any of the preceding claims,
**characterized in that**
a microtome, a cryomicrotome, an ultramicrotome, an ultracryomicrotome, and/or a vibratome is used to separate the embedded supports.

12. The method according to any of the preceding claims,
**characterized in that**
detection molecules immobilized on the first support are crosslinked with each other prior to and/or during and/or subsequent to the removal of the first support by incubation with formaldehyde, glutaraldehyde, glacial acetic acid, bivalent coupling reagents, and/or by polyvalent coupling reagents, particularly oxidized dextran, tosyl-activated dextran, polylysine, proteins, activated proteins, activated polylysine, activated polycarbonates and/or polyethylene glycols.

## Revendications

1. Procédé de préparation d'un lot ordonné d'échantillons pour détecter des constituants dans un échantillon biologique,
**caractérisé en ce**
- **qu'**on fixe les molécules de détection par un procédé de transfert choisi dans le groupe comprenant l'électrobuvardage, le transfert capillaire, l'aspersion et la surimpression sur un ou plusieurs premiers supports, le premier support comportant une membrane,
- on incorpore le ou les premier(s) support(s) dans un matériau,
- on fait durcir le matériau,
- on sépare le(s) premier(s) support(s) incorporé(s) en fractions coupées verticalement,
- on applique au moins une fraction coupée sur un second support,
- on met en solution ou on détache le matériau durci de la fraction appliquée avec un solvant et/ou le(s) premiers support(s) complètement ou partiellement et on obtient le lot ordonné d'échantillons.

2. Procédé selon la revendication 1,
**caractérisé en ce**
**qu'**on incorpore au moins deux premiers supports dans le matériau par superposition.

3. Procédé selon la revendication 1 ou 2,
**caractérisé en ce**
**qu'**en tant que molécules de détection, on utilise des micro-organismes, des extraits de cellules, des ligands, des antigènes, des anticorps, des récepteurs, des acides nucléiques, des lectines, des protéines, des peptides, des glycopeptides, des hydrates de carbone, des acides nucléiques et/ou des lipides.

4. Procédé selon l'une des revendications précédentes,
**caractérisé en ce**
**qu'**on fixe les molécules de détection dans plusieurs lignes sur le premier support.

5. Procédé selon l'une des revendications précédentes,
**caractérisé en ce**
**qu'**on transfère les molécules de détection par transfert de Western et/ou Southern à partir d'un gel sur le premier support.

6. Procédé selon l'une des revendications précédentes,
**caractérisé en ce**
**qu'**en tant que premiers supports, on utilise des supports comprenant de la nitrocellulose, du polyvinylidène difluorure (PVDF), de l'acétate de cellulose, un mélange d'esters de cellulose, du polytétrafluoroéthylène (PTFE), du polyamide, de la cellulose régénérée, du polycarbonate, du polyester, du polysulfone, du polyacrylamide, de l'agarose, du nylon et/ou du polybrène.

7. Procédé selon l'une des revendications précédentes,
**caractérisé en ce**
**qu'**en tant que matériau gélifiant, on utilise des hydrocarbures aliphatiques saturés, notamment de la paraffine, de la résine d'enrobage, des solutions de collagène, des solutions aqueuses, des solutions de protéines se réticulant, des hydrates de carbone, des acides nucléiques, des polymères et/ou de l'agarose.

8. Procédé selon l'une des revendications précédentes,
**caractérisé en ce**
**qu'**en tant que seconds supports, on utilise des supports comprenant du métal, du polypropylène, du téflon, du polyéthylène, du polystyrène, du polyester, de la céramique et/ou du verre.

9. Procédé selon l'une des revendications précédentes,
**caractérisé en ce que**
la surface des supports est modifiée.

10. Procédé selon l'une des revendications précédentes,
**caractérisé en ce que**
qu'en tant que solvant, on utilise un solvant comprenant du chloroforme, du méthanol, de l'éthanol, de l'amylacétate, de l'alcool amylique, du cyclohexanone, du diméthylsulfoxyde, du diéthylacétamide, du diméthylformamide, de l'acétone, de l'acétonitrile, de l'isopropylacétate, du chlorure de méthylène, du méthylcétone, du méthylisobutylcétone, du cellosolve, du tétrahydrofurane, du méthylacétate, de la pyridine, du butylacétate, du dioxane, de l'isopropylacétate, de l'éthylacétate, du diméthylacétamide, de l'acide trifluoroacétique, un agent oxydant, des acides, des bases et/ou des enzymes.

11. Procédé selon l'une des revendications précédentes,
**caractérisé en ce que**,
pour séparer les supports imprégnés, on utilise un microtome, un cryomicrotome, un ultramicrotome, un cryo-ultramicrotome et/ou un microtome vibrant.

12. Procédé selon l'une des revendications précédentes,
**caractérisé en ce**
les molécules de détection fixées sur le premier support sont réticulées par incubation avec du formaldéhyde, du glutaraldéhyde, de l'acide acétique glacial, des réactifs de couplage bivalents et/ou par des réactifs de couplage polyvalents, notamment du dextran oxydé, du dextran tosyl-activé, de la polylysine, des protéines activées, de la polylysine activée, des polycarbonates et/ou des polyéthylène glycols activés, avant et/ou pendant et/ou après le détachement du premier support.
